# EUROPÄISCHE PATENTSCHRIFT

(11) **EP 3 093 046 B1**
(45) Veröffentlichungstag und Bekanntmachung des Hinweises auf die Patenterteilung: **05.06.2019**
(21) Anmeldenummer: 16163487.8
(22) Anmeldetag: 01.04.2016
(51) Int. Cl.: A61Q 5/08, A61Q 5/10, A61K 8/73, A61K 8/23

(54) **MITTEL ZUR SCHONENDEN OXIDATIVEN AUFHELLUNG VON KERATINHALTIGEN FASERN**
MEANS FOR GENTLE OXIDATIVE BRIGHTENING OF KERATIN FIBERS
MOYEN D'ECLAIRCISSEMENT DOUX PAR OXYDATION DE FIBRES CONTENANT DE LA KERATINE

(30) Priorität: 12.05.2015 DE 102015208788
(43) Veröffentlichungstag der Anmeldung: 16.11.2016
(73) Patentinhaber: Henkel AG & Co. KGaA, 40589 Düsseldorf (DE)
(72) Erfinder: ANDERHEGGEN, Bernd, 41189 Mönchengladbach (DE); GOUTSIS, Konstantin, 41363 Jüchen (DE)

(56) Entgegenhaltungen:
- EP-A1- 2 471 502
- WO-A1-2015/028018
- US-B1- 6 302 920

## Beschreibung

Die vorliegende Erfindung betrifft die Verwendung zweier ausgewählter Inhaltsstoffe in Mitteln zur oxidativen Farbveränderung im Bereich der Kosmetik, die sich besonders zum Aufhellen keratinischer Fasern, insbesondere menschlicher Haare, eignen.

Die Veränderung von Form und Farbe der Haare stellt einen wichtigen Bereich der modernen Kosmetik dar. Neben der Färbung ist das Aufhellen der eigenen Haarfarbe bzw. das Blondieren der ganz spezielle Wunsch vieler Verbraucher, da eine blonde Haarfarbe als attraktiv und in modischer Hinsicht erstrebenswert betrachtet wird. Für diesen Zweck sind im Markt verschiedene Blondiermittel mit unterschiedlicher Blondierleistung erhältlich.

Die in Blondiermitteln enthaltenen Oxidationsmittel sind in der Lage, die Haarfaser durch die oxidative Zerstörung des haareigenen Farbstoffes Melanin aufzuhellen. Für einen moderaten Blondiereffekt genügt der Einsatz von Wasserstoffperoxid - gegebenenfalls unter Einsatz von Ammoniak oder anderen Alkalisierungsmitteln - als Oxidationsmittel allein, für das Erzielen eines stärkeren Blondiereffektes wird üblicherweise eine Mischung aus Wasserstoffperoxid und Peroxodisulfatsalzen und/oder Peroxomonosulfatsalzen eingesetzt.

Aus Stabilitätsgründen werden handelsübliche Blondiermittel gewöhnlich in zwei getrennt voneinander verpackten Zubereitungen angeboten, die unmittelbar vor der Anwendung zu einer fertigen Anwendungszubereitung vermischt werden. Üblicherweise bestehen handelsübliche Blondiermittel aus einer flüssigen Oxidationsmittelzubereitung und einem Pulver, das feste Oxidationsmittel enthält.

Zum Blondieren wird das Pulver mit den festen Oxidationsmitteln unmittelbar vor der Anwendung mit der Wasserstoffperoxid-Lösung vermischt. Diese Mischung wird dann auf das Haar aufgebracht und nach einer bestimmten Einwirkzeit wieder ausgespült. Um eine ausreichenden Blondierwirkung zu erzielen, sind derartige Mittel üblicherweise stark alkalisch eingestellt, der pH-Wert liegt dabei zwischen 9 und 10,5. Derart hohe pH-Werte sind erforderlich, um eine Öffnung der äußeren Schuppenschicht (Cuticula) zu gewährleisten und somit eine Penetration der aktiven Spezies (Wasserstoffperoxid und Persulfate) ins Haar zu ermöglichen.

In menschlichen Haaren können sich Spurenelemente, wie zum Beispiel Kupfer, Eisen und Zink anreichern. Besonders blondierte Haare oder geschädigte Haare zeigen eine verstärkte Affinität, Spurenelemente aufzunehmen.

Die Kumulation von Metallionen in den Haarfasern kann auch durch das Trinkwasser erfolgen. In einigen südeuropäischen Ländern als auch in einigen Regionen der USA wird Wasser mit hohen Mengen an Metallionen für die tägliche Körperhygiene verwendet. Aber auch gewisse Lebensumstände, wie die notwendige Einnahme von Medikamenten, wie Cisplatin bei einer Krebstherapie, erhöhen den Metallgehalt im Körper. Die ärztliche Verschreibung von Eisenpräparaten zur Einnahme während der Schwangerschaft oder bei verstärkter Regelblutung als auch Zinkpräparate als Mittel gegen Dermatitis ist gängige Praxis. Die tägliche Einnahme von Nahrungsergänzungsmitteln auf Basis von Mineralstoffen gehört für einige Menschen zum gesunden Lebensstil.

Alle diese Faktoren sind potentielle Quellen, die zu einer Kumulation von Spurenelementen/Metallionen über das erforderliche essentielle Maß im Körper und letztendlich in den Haaren führen können.

Die in den Haarfasern angereicherten Metallionen können die Zersetzung des Wasserstoffs und der Persulfate katalysieren, was zu einer unerwünschten Hitzeentwicklung führt.

Die US 5,635,167 schlägt deshalb ein Verfahren zur Entfernung von Metallionen wie Kupfer oder Eisen vor, bei dem eine Lösung, die 4 bis 25 Gew.-% eines Chelatisierungsmittels und einen pH-Wert von 4 bis 9 aufweist, eingesetzt wird.

Die EP 1714634 A1 beschreibt Haarbehandlungs-Kit zum Färben von menschlichen Haaren, umfassend ein erstes Kompartiment, welches einen Komplexbildner enthält, und ein zweites Kompartiment, welches Mittel zum Färben enthält. Auch hier sollen unerwünschte Reaktionen an und mit Haaren, die zu einer unerwünschten Erwärmung führen, verhindert werden.

Aus der EP 2471502 A1 ist ein Bleich- und/oder Aufhellmittel für menschliches Haar, enthaltend mindestens eine Verbindung mit Bleich- und/oder Aufhellwirkung und eine Polyhydroxycarbonsäure als Komplexbildner, bekannt.

Es hat sich aber gezeigt, dass es auch bei Blondiermitteln, die Komplexbildner enthalten, noch zu einer unerwünscht hohen Wärmeentwicklung kommen kann

Es besteht weiterhin Bedarf, Blondiermittel bereit zu stellen, bei deren Anwendung die Wärmeentwicklung und die dadurch verursachte Schädigung von Haar und/oder Kopfhaut reduziert werden.

Diese Aufgabe wird gelöst durch die Verwendung eines Polysaccharids und eines Salzes der Ethylendiamintetraessigsäure (EDTA) in einer Zubereitung (A), welche Bestandteil eines Mittels zum Aufhellen keratinischer Fasern ist, enthaltend mindestens zwei getrennt voneinander verpackte Zubereitungen (A) und (B) sowie gegebenenfalls eine weitere getrennt von (A) und (B) verpackte Zubereitung (C), die unmittelbar vor der Anwendung zu einer Anwendungsmischung vermischt werden, wobei
i. Zubereitung (A) mindestens ein Persulfat enthält und
ii. Zubereitung (B) fließfähig ist und mindestens ein Oxidationsmittel enthält,
zur Verminderung von Haar- und/oder Kopfhautschädigungen und/oder
zur Verringerung der entstehenden Wärmemenge
beim Aufhellen keratinischer Fasern.

Unter keratinischen Fasern oder auch Keratinfasern sind dabei Pelze, Wolle, Federn und insbesondere menschliche Haare zu verstehen. Obwohl die Mittel in erster Linie zum Aufhellen von Keratinfasern geeignet sind, steht prinzipiell einer Verwendung auch auf anderen Gebieten nichts entgegen.

Die Zubereitungen (A) sind bevorzugt pulverförmig. Dabei können Pulver aus festen Bestandteilen mit unterschiedlichen Korngrößen eingesetzt werden. Üblicherweise kann es bevorzugt sein, wenn die Pulver jedoch eine möglichst homogene Korngröße aufweisen, insbesondere um eine einheitliche Dispersion bzw. Auflösung der Pulver in den Zubereitungen (B) zu erleichtern.

Die Zubereitungen (A) können die Wirkstoffe in einem festen kosmetischen Träger enthalten. Ein fester kosmetischer Träger kann Salze der Kieselsäure, insbesondere Salze der Silicate und Metasilicate mit Ammonium, Alkalimetallen sowie Erdalkalimetallen enthalten. Insbesondere Metasilicate, die sich gemäß Formel (SiO₂)ₙ(M₂O)ₘ , wobei M für ein Ammoniumion, ein Alkalimetall oder ein halbes Stöchiometrieäquivalents eines Erdalkalimetalls steht, durch das Verhältnis zwischen n und m von ≤ 1 auszeichnen und sich als kettenförmige polymere Strukturen des Anions [SiO₃]²⁻ auffassen lassen, können bevorzugt eingesetzt werden. Natriummetasilicat der Formel [Na₂SiO₃]_{∞}, ist dabei besonders bevorzugt. Gleichfalls bevorzugt sind solche Silicate, die aus einem Silicat der Formel (SiO₂)ₙ(Na₂O)ₘ(K₂O)ₚ gebildet werden, wobei n für eine positive rationale Zahl und m und p unabhängig voneinander für eine positive rationale Zahl oder für 0 stehen, mit den Maßgaben, dass mindestens einer der Parameter m oder p von 0 verschieden ist und das Verhältnis zwischen n und der Summe aus m und p zwischen 2:1 und 4:1 liegt.

Weiterhin können die festen kosmetischen Träger so genannte Rieselhilfen enthalten, die ein Verklumpen oder Verbacken der Pulver-Bestandteile verhindern sollen. Als solche Rieselhilfen kommen bevorzugt wasserunlösliche, hydrophobierende oder Feuchtigkeit adsorbierende Pulver von Kieselgur, pyrogenen Kieselsäuren, Calciumphosphat, Calciumsilicaten, Aluminiumoxid, Magnesiumoxid, Magnesiumcarbonat, Zinkoxid, Stearaten, Fettaminen und dergleichen in Frage.

Schließlich können die festen kosmetischen Träger noch zusätzlich ein Entstaubungsmittel enthalten, die die Staubbildung der pulverförmigen Bestandteile verhindert. Hierzu können insbesondere inerte Öle eingesetzt werden. Bevorzugt enthalten die festen, kosmetischen Träger als Entstaubungsmittel Esteröle oder Mineralöle, bevorzugt Kohlenwasseröle, wie flüssiges Paraffinöl.

Als ersten wesentlichen Inhaltsstoff enthält die Zubereitung (A) mindestens ein Persulfatsalz. Geeignete Persulfatsalze sind anorganische Peroxoverbindungen. Diese sind bevorzugt ausgewählt aus Ammoniumperoxodisulfat, Alkalimetallperoxodisulfaten, Ammoniumperoxomonosulfat, Alkalimetallperoxomonosulfaten, Alkalimetallperoxodiphosphaten und/oder Erdalkalimetallperoxiden. Besonders bevorzugt sind Ammoniumperoxodisulfat und/oder Alkalimetallperoxodisulfate.

In einer bevorzugten Ausführungsform der vorliegenden Erfindung enthält Zubereitung (A) als Persulfatsalz mindestens ein Peroxodisulfatsalz, insbesondere ausgewählt aus Ammoniumperoxodisulfat und/oder Kaliumperoxodisulfat und/oder Natriumperoxodisulfat.

Weiterhin hat es sich als besonders bevorzugt erwiesen, wenn die Zubereitungen (A) mindestens zwei verschiedene Peroxodisulfate enthalten. Bevorzugte Peroxodisulfatsalze sind dabei Kombinationen von Ammoniumperoxodisulfat mit Kaliumperoxodisulfat und/oder Natriumperoxodisulfat.

Die Zubereitungen (A) enthalten Persulfatsalze vorzugsweise in einer Menge von 0,1 bis 80 Gew.-%, bevorzugt von 2 bis 60 Gew.-%, besonders bevorzugt von 3 bis 50 Gew.-% und insbesondere bevorzugt 5 bis 45 Gew.-%, jeweils bezogen auf das Gesamtgewicht der Zubereitung (A).

Als zweiten wesentlichen Inhaltsstoff enthalten die Zubereitungen (A) ein Polysaccharid. Polysaccharide sind durch Entzug von ungebundenem Wasser in der Lage, die Viskosität einer Flüssigkeit zu erhöhen.

In einer bevorzugten Ausführungsform enthält die Zubereitung (A) ein Cellulosederivat als Polysaccharid. Dieses Cellulosederivat ist vorzugsweise ausgewählt aus der Gruppe bestehend aus Methylcellulose, Ethylcellulose, Propylcellulose, Methylethylcellulose, Carboxymethylcellulose, Ethylcarboxymethylcellulose, Hydroxyethylcellulose, Hydroxypropylcellulose, Carboxymethylhydroxyethylcellulose, Methylhydroxyethylcellulose Hydroxypropylmethylcellulose, Ethylhydroxyethylcellulose, Methylethylhydroxyethylcellulose und Mischungen dieser Verbindungen. Es hat sich gezeigt, dass insbesondere der Einsatz von Carboxymethylcellulose als Polysaccharid zu einer starken Verminderung der bei der Aufhellung von keratinischen Fasern, die mit Metallionen angereichert sind, entstehenden Wärmemenge führt.

Carboxymethylcellulosen (CMC) sind Celluloseether, bei denen ein Teil der Hydroxygruppen als Ether mit einer Carboxymethyl-Gruppe (-CH₂-COOH) verknüpft sind. Carboxymethylcellulosen sind in der Säureform unlöslich in Wasser. Entsprechend wird ganz besonders bevorzugt, das Natriumsalz einer Carboxymethylcellulose als Polysaccharid in der Zubereitung (A) eingesetzt.

Geeignete Natrium-Carboxymethylcellulosen sind beispielsweise unter den Bezeichnungen "Aqualon" oder "Blanose" von Ashland Inc. oder unter der Bezeichnung "Cekol®" von CP Kelco erhältlich.

Es ist ferner bevorzugt, dass Zubereitung (A) - bezogen auf ihr Gewicht - Polysaccharid in einer Menge von 0,5 bis 5,0 Gew.-%, bevorzugt von 1,0 bis 4,0 Gew.-% und besonders bevorzugt von 1,5 bis 3,0 Gew.-% enthält.

Als weiteren zwingenden Inhaltsstoff enthält Zubereitung (A) ein Salz der Ethylendiamintetraessigsäure (EDTA).Als besonders vorteilhaft bezüglich der Verminderung der bei der Aufhellung von keratinischen Fasern, die mit Metallionen angereichert sind, entstehenden Wärmemenge hat sich der Einsatz eines Natriumsalzes der Ethylendiamintetraessigsäure (EDTA) in der Zubereitung (A) erwiesen. Geeignete EDTA-Salze umfassen beispielsweise Dinatriumethylendiamintetraacetat (Na₂H₂EDTA), Tetranatriumethylendiamintetraacetat (Na₄EDTA) und Calciumdinatriumethylendiamintetraacetat (CaNa₂EDTA), wobei Dinatriumethylendiamintetraacetat bevorzugt ist.

In einer ganz besonders bevorzugten Ausführungsform enthält Zubereitung (A) - bezogen auf ihr Gewicht - das Salz der Ethylendiamintetraessigsäure (EDTA) in einer Menge größer 0,6 Gew.-%, bevorzugt in Mengen von 0,8 bis 2,5 Gew.-%, mehr bevorzugt von 1,0 bis 2,2 Gew.-% und ganz besonders bevorzugt von 1,2 bis 2,0 Gew.-%.

Ohne an diese Theorie gebunden sein zu wollen werden durch die Zugabe eines Polysaccharids die Inhaltsstoffe des anwendungsbereiten Mittels immobilisiert und insbesondere die Metallionen katalysierte Zersetzung des Wasserstoffperoxids gegenüber der Komplexierung der Metallionen durch das EDTA-Salz verlangsamt.

Die Zubereitungen (B) und gegebenenfalls (C) enthalten die Wirkstoffe in einem fließfähigen, kosmetischen Träger. Die Grundlage des fließfähigen, kosmetischen Trägers ist dabei bevorzugt wässrig oder wässrig-alkoholisch. Zum Zwecke der Haarbleiche sind solche Träger beispielsweise Gele oder auch tensidhaltige schäumende Lösungen, wie beispielsweise Shampoos, Schaumaerosole oder andere Zubereitungen, die für die Anwendung auf dem Haar geeignet sind. Ein bevorzugter, fließfähiger Träger enthält im Sinne der Erfindung mindestens 40 Gew.-%, insbesondere mindestens 50 Gew.-% Wasser. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, insbesondere Ethanol bzw. Isopropanol, zu verstehen. Die Zubereitungen (B) und gegebenenfalls (C) können jeweils zusätzlich weitere organische Lösungsmittel, wie beispielsweise Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether enthalten. Bevorzugt sind dabei alle wasserlöslichen organischen Lösungsmittel.

Die Zubereitungen (B) enthalten Wasserstoffperoxid als Oxidationsmittel.

Die Konzentration einer Wasserstoffperoxid-Lösung in der Zubereitung (B) wird einerseits von den gesetzlichen Vorgaben und andererseits von dem gewünschten Effekt bestimmt.

Vorzugsweise enthalten die Zubereitungen (B) bezogen auf ihr Gewicht Wasserstoffperoxid in Mengen von 0,5 bis 30 Gew %, bevorzugt von 1 bis 20 Gew.-%, besonders bevorzugt von 5 bis 15 Gew.-% und insbesondere bevorzugt von 6 bis 12 Gew.-%, explizit 6, 7, 8, 9, 10, 11 oder 12 Gew.-%.

Bevorzugte anwendungsbereite Mittel sind dadurch gekennzeichnet, dass sie, bezogen auf das Gesamtgewicht des anwendungsbereiten Mittels, 0,01 bis 12 Gew.-%, vorzugsweise 0,1 bis 10 Gew.-%, besonders bevorzugt 3 bis 9 Gew.-% Wasserstoffperoxid enthalten.

Die anwendungsbereiten Mittel zum Aufhellen keratinischer Fasern werden unmittelbar vor der Anwendung auf dem Haar durch Mischen der zwei Zubereitungen (A) und (B) und gegebenenfalls einer dritten Zubereitung (C) und/oder weiteren Zubereitungen hergestellt. Die Konsistenz der anwendungsbereiten Mittel reicht von fließfähig bis streichfest.

Die viskosen Eigenschaften von Zubereitung (B) sind von Bedeutung für ihre gute Mischbarkeit und hohe Stabilität. Die Zubereitungen (B) weisen daher bevorzugt eine Viskosität von 1.000 mPa·s bis 50.000 mPa·s, vorzugsweise von 5.000 mPa·s bis 45.000 mPa·s und besonders bevorzugt von 7.000 mPa·s bis 40.000 mPa·s bei Messungen mit einem Rotationsviskosimeter von Brookfield, Spindelgröße 4, bei 25°C und 4 Upm, auf. Die fertig angemischten und anwendungsbereiten Mittel weisen vorzugsweise eine Viskosität von 10.000 mPa·s bis 100.000 mPa·s und besonders bevorzugt von 18.000 mPa·s bis 80.000 mPa·s bei Messungen mit einem Rotationsviskosimeter von Brookfield, Spindelgröße 5, bei 25°C und 4 Upm auf.

Die Mittel können weitere Wirk- und Hilfsstoffe beinhalten. Diese werden nachfolgend beschrieben.

Es kann von Vorteil sein, wenn Zubereitung (B) mindestens ein nichtionisches Tensid, bevorzugt mindestens einen ethoxylierten Fettalkohol mit 40 bis 60 Ethylenoxideinheiten enthält. Darunter ist ein Anlagerungsprodukt von Ethylenoxid an einen Fettalkohol zu verstehen. Fettalkohole sind dabei gesättigte und ungesättigte Alkohole mit 12 bis 24 C-Atomen, welche linear oder verzweigt sein können. Die Molmenge an Ethylenoxid, die pro Mol Fettalkohol eingesetzt wurde, bezeichnet dabei den Ethoxylierungsgrad verstanden. Als nichtionisches Tensid eignen sich dabei insbesondere Ethylenoxid-Anlagerungsprodukte an Octylalkohol (Caprylalkohol), Nonylalkohol (Pelargonylalkohol), Undecylalkohol, Undec-10-en-1-ol, Dodecylalkohol (Laurylalkohol), 2,6,8-Trimethyl-4-nonanol (Isolaurylalkohol), Tridecylalkohol, Tetradecylalkohol (Myristylalkohol), Pentadecylalkohol, Hexadecylalkohol (Cetyl-/ Palmitylalkohol), Heptadecylalkohol, Octadecylalkohol (Stearylalkohol), Isostearylalkohol, (9Z)-Octadec-9-en-1-ol (Oleylalkohol), (9E)-Octadec-9-en-1-ol (Elaidylalkohol), (9Z,12Z)-Octadeca-9,12-dien-1-ol (Linoleylalkohol), (9Z,12Z,15Z)-Octadeca-9,12,15-trien-1-ol (Linolenylalkohol), Nonadecan-1-ol (Nonadecylalkohol), Eicosan-1-ol (Eicosylalkohol / Arachylalkohol), (9Z)-Eicos-9-en-1-ol (Gadoleylalkohol), (5Z,8Z,11Z,14Z)-Eicosa-5,8,11,14-tetraen-1-ol (Arachidonalkohol), Heneicosylalkohol, Docosylalkohol (Behenylalkohol), (13Z)-Docos-13-en-1-ol (Erucylalkohol) oder (13E)-Docosen-1-ol (Brassidylalkohol). Es ist ebenfalls möglich, Gemische von Fettalkoholen, die durch gezielte Mischung oder auch durch Gewinnungsverfahren als solche anfallen, einzusetzen. Beispiele sind Cocosalkohol (Mischung aus C₈-C₁₈-Fettalkoholen) oder Cetearylalkohol (1:1-Mischung aus C₁₆- und C₁₈-Fettalkoholen).

Bevorzugt sind Ethoxylierungsgrade von 20 bis 60. Bevorzugte nichtionische Tenside vom Typ ethoxylierter Fettalkohol sind Ceteareth-20 und Ceteareth-50.

Weiterhin ist die Einstellung des pH-Wertes des fertig angemischten und anwendungsbereiten Mittels von Bedeutung für die Aufhellleistung. Bevorzugt sind fertig angemischte und anwendungsbereite Mittel, deren pH-Wert zwischen 9 und 12 liegt.

Weiterhin können die Mittel deshalb Alkalisierungsmittel enthalten. Bevorzugte Alkalisierungsmittel sind beispielsweise Ammoniak, Alkanolamine, basische Aminosäuren, sowie anorganische Alkalisierungsmittel wie (Erd-)Alkalimetallhydroxide, (Erd-)Alkalimetallmetasilikate, (Erd-)Alkalimetallsilikate, (Erd-) Alkalimetallphosphate und (Erd-)Alkalimetallhydrogenphosphate. Als Metallionen dienen bevorzugt Lithium, Natrium und/oder Kalium. Bevorzugte Alkalisierungsmittel sind (Erd-)Alkalimetallmetasilikate und (Erd-)Alkalimetallsilikate.

Geeignete, anorganische Alkalisierungsmittel sind bevorzugt ausgewählt aus Natriumhydroxid, Kaliumhydroxid, Calciumhydroxid, Bariumhydroxid, Natriumphosphat, Kaliumphosphat, Natriumsilicat, Kaliumsilicat, Magnesiumsilicat, Natriumcarbonat und Kaliumcarbonat. Besonders bevorzugt sind Natriumhydroxid und/oder Kaliumhydroxid.

Alkanolamine als Alkalisierungsmittel werden bevorzugt ausgewählt aus primären, sekundären oder tertiären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methyl-propanol, 2-Amino-2-methylbutanol, 3-Amino-2-methylpropan-1-ol, 1-Amino-2-methylpropan-2-ol, 3-Aminopropan-1,2-diol, 2-Amino-2-methylpropan-1,3-diol, 2-Amino-2-ethyl-1,3-propandiol, N,N-Dimethylethanolamin, Methylglucamin, Triethanolamin, Diethanolamin und Triisopropanolamin. Insbesondere bevorzugte Alkanolamine sind Monoethanolamin, 2-Amino-2-methyl-propanol und Triethanolamin.

Basische Aminosäuren als Alkalisierungsmittel werden bevorzugt ausgewählt aus der Gruppe, die gebildet wird aus L-Arginin, D-Arginin, D/L-Arginin, L-Lysin, D-Lysin, D/L-Lysin, L-Ornithin, D-Ornithin, D/L- Ornithin, L-Histidin, D-Histidin und/oder D/L-Histidin. Besonders bevorzugt werden L-Arginin, D-Arginin und/oder D/L-Arginin als ein Alkalisierungsmittel eingesetzt.

Die Zubereitungen (A) und (B) können mit weiteren separat verpackten Zubereitungen unmittelbar vor der Anwendung zu einer Anwendungsmischung vermischt werden.

In einer bevorzugten Ausführungsform der Erfindung enthält das Mittel zusätzlich mindestens eine weitere getrennt von Zubereitungen (A) und (B) verpackte Zubereitung (C), wobei Zubereitung (C) mindestens ein Alkalisierungsmittel enthält.

Unabhängig davon, ob Zubereitung (C) und/oder Zubereitung (B) und/oder weitere Zubereitungen Alkalisierungsmittel enthalten, sind, wenn Alkalisierungsmittel zum Einsatz kommen, solche Mittel bevorzugt, die Alkalisierungsmittel in einer Menge von 0,05 bis 20 Gew.-%, insbesondere von 0,5 bis 10 Gew.-%, jeweils bezogen auf das Gesamtgewicht des gesamten Mittels, enthalten.

Zur weiteren Steigerung der Aufhellleistung kann Zubereitung (C) zusätzlich als Bleichverstärker eine Silicium-haltige Verbindung zugesetzt werden. Diese wird bevorzugt ausgewählt aus der Gruppe, die gebildet wird aus Kieselsäure, Alkalimetallsilicaten und Erdalkalimetallsilicaten.

Besonders vorteilhaft ist es, wenn die Aufhell- bzw. Blondiermittel zur Mattierung von unerwünschten Restfarbeindrücken, insbesondere im rötlichen oder bläulichen Bereich, bestimmte, direktziehende Farbstoffe der komplementären Farben enthalten. Dabei handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole. Direktziehende Farbstoffe sind als anionische, kationische und nichtionische direktziehende Farbstoffe bekannt. Die direktziehenden Farbstoffe werden jeweils bevorzugt in einer Menge von 0,001 bis 2 Gew.-%, bezogen auf die gesamte Anwendungszubereitung, eingesetzt.

Ferner können die Mittel weitere Wirk-, Hilfs- und Zusatzstoffe enthalten, wie beispielsweise nichtionische Polymere, kationische Polymere, anionische Polymere, zwitterionische und amphotere Polymere, Strukturanten, weitere haarkonditionierende Verbindungen, weitere faserstrukturverbessernde Wirkstoffe, weitere Tenside, Farbstoffe zum Anfärben des Mittels, Antischuppenwirkstoffe, Aminosäuren, Oligopeptide, Proteinhydrolysate auf tierischer und/oder pflanzlicher Basis, Lichtschutzmittel, UV-Blocker, Vitamine, Provitamine, Vitaminvorstufen, Pflanzenextrakte, Fette, Wachse, Quell- und Penetrationsstoffe, Trübungsmittel, Perlglanzmittel, Verdickungsmittel und Pigmente.

Die anwendungsbereiten Mittel werden unmittelbar vor der Anwendung auf dem Haar durch Mischen der zwei Zubereitungen (A) und (B) und gegebenenfalls einer dritten Zubereitung (C) und/oder weiteren Zubereitungen hergestellt. Bei anwendungsbereiten Mitteln, die aus mehr als zwei Zubereitungen zu einer fertigen Anwendungsmischung vermischt werden, kann es unerheblich sein, ob zunächst zwei Zubereitungen miteinander vermischt werden und anschließend die dritte Zubereitung zugegeben und untergemischt wird, oder ob alle Zubereitungen gemeinsam zusammengeführt und anschließend vermischt werden. Das Vermischen kann durch Verrühren in einer Schale oder einem Becher erfolgen oder durch Schütteln in einem verschließbaren Behälter.

Der Begriff "unmittelbar" ist dabei als Zeitraum von wenigen Sekunden bis eine Stunde, vorzugsweise bis 30 min, insbesondere bis 15 min zu verstehen.

Die Mittel werden in einem Verfahren zum Aufhellen von keratinischen Fasern, insbesondere menschlichen Haaren, angewendet, bei dem das Mittel auf die keratinhaltigen Fasern aufgebracht, für eine Einwirkdauer von 10 bis 60 Minuten auf der Faser belassen und anschließend mit Wasser wieder ausgespült oder mit einem Shampoo ausgewaschen wird.

Bevorzugt beträgt die Einwirkzeit der anwendungsbereiten Aufhellmittel 10 bis 60 min, insbesondere 15 bis 50 min, besonders bevorzugt 20 bis 45 min. Während der Einwirkzeit des Mittels auf der Faser kann es vorteilhaft sein, den Aufhellvorgang durch geringe Wärmezufuhr zu unterstützen. Die Wärmezufuhr kann durch eine externe Wärmequelle, wie mit Hilfe eines Warmluftgebläses, als auch, insbesondere bei einer Haaraufhellung am lebenden Probanden, durch die Körpertemperatur des Probanden erfolgen. Bei letzterer Möglichkeit wird üblicherweise die aufzuhellende Partie mit einer Haube abgedeckt. Eine Einwirkphase bei Raumtemperatur ist bevorzugt.

Nach Ende der Einwirkzeit wird die verbleibende Aufhellzubereitung mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Leitungswasser erfolgen kann, wenn das Aufhellmittel einen stark tensid-haltigen Träger besitzt.

Die folgenden Beispiele sollen den Gegenstand der vorliegenden Erfindung erläutern ohne ihn jedoch zu beschränken.

### Beispiele

**Tabelle 1: Zusammensetzung Zubereitungen (A) für Blondiermittel aus zwei Zubereitungen**

| Inhaltsstoff | E1 | E2 | V1 |
|---|---|---|---|
| Na₂H₂EDTA | 0,6 | 2,0 | 0,6 |
| Natriumhexametaphosphat | 0,3 | 0,3 | 0,3 |
| Natriumsilikat | 36 | 36 | 36 |
| Aerosil 200 | 0,4 | 0,4 | 0,4 |
| Rohagit S hv | 1,0 | 1,0 | 1,0 |
| Cekol 50000 | 2,0 | 2,0 | -- |
| Ammoniumpersulfat + 0,5 % Kieselsäure | 10,0 | 10,0 | 10,0 |
| Kaliumpersulfat | 32 | 32 | 32 |
| Paraffinum Liquidum | 3,6 | 3,6 | 3,8 |
| Parfüm | 0,5 | 0,5 | 0,5 |
| Dimethicone | 1,5 | 1,5 | 1,5 |
| Polyquaternium-4 | 0,3 | 0,3 | 0,3 |
| Farbstoff | 0,1 | 0,1 | 0,1 |
| Magnesiumcarbonat | ad 100 | ad 100 | ad 100 |

| | | | |
|---|---|---|---|
| *eingesetzte Rohstoffe: Aerosil 200 (INCI Bezeichnung: Silica (Evonik Degussa)), Rohagit S hv (INCI Bezeichnung: Acrylates Copolymer (Evonik Röhm)), Cekol 50000 (INCI Bezeichnung: Cellulose Gum (CP Kelco)) | | | |

**Tabelle 2: Zusammensetzung einer Zubereitung (B) für Blondiermittel aus zwei Zubereitungen**

| Inhaltsstoff | B |
|---|---|
| Kalilauge 45 % techn. | 0,1 |
| Dipicolinsäure | 0,1 |
| Dinatriumpyrophosphat | 0,03 |
| HEDP 60% | 1,5 |
| Wasserstoffperoxid 50 % | 18 |
| PEG-40 Castor Oil | 0,7 |
| Cetearyl Alcohol | 4,0 |
| Wasser, vollentsalzt | ad 100 |

Zur Herstellung eines Blondiermittels wurde jeweils eine der Zubereitungen (A) im Verhältnis 1:2 mit der Zubereitung (B) gemischt.

### Haarschädigungsuntersuchungen mittels NIR-Analytik

Haare können durch oxidative Behandlung wie eine oxidative Aufhellung geschädigt werden. Cystin ist ein Bestandteil des menschlichen Haares. Die in Cystin enthaltene Disulfid-Brücke kann bei einer Haarbehandlung oxidativ gebrochen und das resultierende Thiol durch weitere Oxidation in die Sulfonsäure-Gruppe der Cysteinsäure überführt werden. Somit verändert sich das Haar hinsichtlich seines Cysteinsäuregehaltes.

Die Abnahme an Cystin und die Zunahme an Cysteinsäure stellt daher einen Indikator für die Schädigung des Haares dar, die mit NIR-Analysen bestimmt werden kann.

Zur Messung der Haarschädigung wurde der Cysteinsäurewert jeder behandelten Haarsträhne mittels quantitativer NIR-Spektroskopie bestimmt. Die Aufnahme der Spektren erfolgte mit einem MPA™ FT-NIR Spektrometer der Firma Bruker Optik GmbH. Der Infrarot-Bereich umfasst den Wellenzahlbereich von 12500 cm⁻¹ bis 4000 cm⁻¹ und ist für Oberton- und Kombinationsschwingungen von z. B. CH-, OH- und NH-Gruppen charakteristisch.

Die Messung der Proben wurde mit dem Intetrationskugelmodul an sechs verschiedenen Probenpositionen in diffuser Reflexion durchgeführt. Für die Analyse der gemessenen NIR-Spektren wurde der Wellenzahlbereich von 7300 cm⁻¹ bis 4020 cm⁻¹ gewählt.

Die NIR Spektren von Cystin zeigen im Wellenzahlbereich von 6200 cm⁻¹ bis 5500 cm⁻¹ charakteristische Absorptionsbanden. Verändert sich das Haar durch stärkere Schädigung (d.h. nimmt der Cysteinsäuregehalt in den Haaren zu) wirkt sich dies im NIR-Spektrum auf die für Cysteinsäure charakteristischen Banden bei 5020 cm⁻¹ bis 4020 cm⁻¹ aus. Die quantitative Auswertung der NIR- Spektren erfolgte rechnergestützt.

Zur Schädigung und Dotierung der Haarsträhnen wurden diese zwei Mal vorblondiert und anschließend wurde die trockene Haarsträhne für 1 Minute in 20 ml einer Kupferionen-haltigen Lösung (Gehalt an Cu²⁺ = 200 ppm) getaucht. Das Dotierungsverfahren wurde insgesamt drei Mal durchgeführt.

Die eingesetzten Haarsträhnen waren:

| | |
|---|---|
| H1: Kerling Euronaturhaar 6-0 | unbehandelt |
| H2: Kerling Euronaturhaar 6-0 | zwei Mal vorblondiert mit einem Blondiermittel auf Basis der Zubereitung (A)-V1, drei Mal dotiert mit Kupferionen und ein Mal nachblondiert mit einem Blondiermittel auf Basis der Zubereitung (A)-V1 |
| H3: Kerling Euronaturhaar 6-0 | zwei Mal vorblondiert mit einem Blondiermittel auf Basis der Zubereitung (A)-V1, drei Mal dotiert mit Kupferionen und drei Mal nachblondiert mit einem Blondiermittel auf Basis der Zubereitung (A)-V1 |
| H4: Kerling Euronaturhaar 6-0 | zwei Mal vorblondiert mit einem Blondiermittel auf Basis der Zubereitung (A)-E2, drei Mal dotiert mit Kupferionen und ein Mal nachblondiert mit einem Blondiermittel auf Basis der Zubereitung (A)-E2 |
| H5: Kerling Euronaturhaar 6-0 | zwei Mal vorblondiert mit einem Blondiermittel auf Basis der Zubereitung (A)-E2, drei Mal dotiert mit Kupferionen und drei Mal nachblondiert mit einem Blondiermittel auf Basis der Zubereitung (A)-E2 |

Es wurden jeweils 3 Haarsträhnen hergestellt und vermessen.

**Tabelle 3: NIR-Analysenwerte:**

| Haarsträhne | Cysteinsäure [Gew.-%] |
|---|---|
| H1 | 0,4 |
| H2 | 11,7 |
| H3 | 12,7 |
| H4 | 10,2 |
| H5 | 12,4 |

Die Ergebnisse zeigen deutlich, dass das erfindungsgemäße Blondiermittel weniger haarschädigend ist.

Außerdem wurde die maximale Temperatur sowie die innerhalb von 45 Minuten nach Auftragung des jeweiligen Blondiermittels entstehende Gesamtwärmemenge bei der Blondierung von Haaren bestimmt. Diese Bestimmung erfolgte auch an Haarsträhnen, die zwei Mal vorblondiert und drei Mal mit Kupferionen dotiert worden waren. Die Vorblondierung erfolgte jeweils mit dem Blondiermittel, welches auch bei der Blondierung eingesetzt wurde.

**Tabelle 4: maximale Temperatur und Wärmemenge bei Blondierverfahren**

| Blondiermittel (Mischungsverhältnis) | Tₘₐₓ [°C] | Wärmemenge |
|---|---|---|
| (A)-V1 + (B) (1 : 2) | 57 | 251 |
| (A)-E1 + (B) (1 : 2) | 45 | 319 |
| (A)-E2 + (B) (1 : 2) | 44 | 241 |

Zur Bestimmung der Wärmemenge wurde die Temperatur bei der Blondierung über einen Zeitraum 45 Minuten bestimmt und graphisch gegen die Zeit aufgetragen. Anschließend wurde die Fläche unter den erhaltenden Kurven bis zu einer Temperatur von 30 °C bestimmt. Diese ist proportional zur entstandenen Wärmemenge.

## Patentansprüche

1. Verwendung eines Polysaccharids und eines Salzes der Ethylendiamintetraessigsäure (EDTA) in einer Zubereitung (A), welche Bestandteil eines Mittels zum Aufhellen keratinischer Fasern ist, enthaltend mindestens zwei getrennt voneinander verpackte Zubereitungen (A) und (B) sowie gegebenenfalls eine weitere getrennt von (A) und (B) verpackte Zubereitung (C), die unmittelbar vor der Anwendung zu einer Anwendungsmischung vermischt werden, wobei
i. Zubereitung (A) mindestens ein Persulfat enthält und
ii. Zubereitung (B) fließfähig ist und mindestens ein Oxidationsmittel enthält,
zur Verminderung von Haar- und/oder Kopfhautschädigungen und/oder
zur Verringerung der entstehenden Wärmemenge
beim Aufhellen keratinischer Fasern.

2. Verwendung nach Anspruch 1, **dadurch gekennzeichnet, dass** Zubereitung (A) als Polysaccharid ein Cellulosederivat ausgewählt aus der Gruppe bestehend aus Methylcellulose, Ethylcellulose, Propylcellulose, Methylethylcellulose, Carboxymethylcellulose, Ethylcarboxymethylcellulose, Hydroxyethylcellulose, Hydroxypropylcellulose, Carboxymethylhydroxyethylcellulose, Methylhydroxyethylcellulose Hydroxypropylmethylcellulose, Ethylhydroxyethylcellulose, Methylethylhydroxyethylcellulose und Mischungen dieser Verbindungen enthält.

3. Verwendung nach Anspruch 2 **dadurch gekennzeichnet, dass** Zubereitung (A) als Polysaccharid Carboxymethylcellulose enthält.

4. Verwendung nach einem der Ansprüche 1 bis 3, **dadurch gekennzeichnet, dass** Zubereitung (A) ein Natriumsalz der Ethylendiamintetraessigsäure (EDTA) enthält.

5. Verwendung nach Anspruch 4, **dadurch gekennzeichnet, dass** Zubereitung (A) Dinatriumethylendiamintetraacetat (Na₂H₂EDTA) enthält.

6. Verwendung nach einem der Ansprüche 1 bis 5, **dadurch gekennzeichnet, dass** Zubereitung (A) - bezogen auf ihr Gewicht - das Salz der Ethylendiamintetraessigsäure (EDTA) in einer Menge größer 0,6 Gew.-%, bevorzugt in Mengen von 0,8 bis 2,5 Gew.-%, mehr bevorzugt von 1,0 bis 2,2 Gew.-% und ganz besonders bevorzugt von 1,2 bis 2,0 Gew.-% enthält.

7. Verwendung nach einem der Ansprüche 1 bis 6, **dadurch gekennzeichnet, dass** Zubereitung (A) mindestens ein Persulfat, ausgewählt aus Ammoniumperoxodisulfat und/oder Kaliumperoxodisulfat und/oder Natriumperoxodisulfat, enthält.

8. Verwendung nach einem der Ansprüche 1 bis 7, **dadurch gekennzeichnet, dass** Zubereitung (B) bezogen auf ihr Gewicht Wasserstoffperoxid in Mengen von 0,5 bis 30 Gew.-%, vorzugsweise von 1 bis 20 Gew.-%, besonders bevorzugt von 5 bis 15 Gew.-% und insbesondere bevorzugt von 6 bis 12 Gew.-%, enthält.

## Claims

1. The use of a polysaccharide and a salt of ethylenediaminetetraacetic acid (EDTA) in a preparation (A) which is part of an agent for lightening keratin fibers, containing at least two preparations (A) and (B) which are packaged separately from one another and optionally a further preparation (C) which is packaged separately from (A) and (B) and is mixed immediately before application to form an application mixture, wherein
i. preparation (A) contains at least one persulfate and
ii. preparation (B) is flowable and contains at least one oxidizing agent,
for reducing damage to hair and/or the scalp and/or
for reducing the amount of heat generated when lightening keratin fibers.

2. The use according to claim 1, **characterized in that** preparation (A) contains, as the polysaccharide, a cellulose derivative selected from the group consisting of methyl cellulose, ethyl cellulose, propyl cellulose, methyl ethyl cellulose, carboxymethyl cellulose, ethyl carboxymethyl cellulose, hydroxyethyl cellulose, hydroxypropyl cellulose, carboxymethyl hydroxyethyl cellulose, methyl hydroxyethyl cellulose, hydroxypropyl methyl cellulose, ethyl hydroxyethyl cellulose, methyl ethyl hydroxyethyl cellulose and mixtures of these compounds.

3. The use according to claim 2, **characterized in that** preparation (A) contains carboxymethyl cellulose as the polysaccharide.

4. The use according to one of claims 1 to 3, **characterized in that** preparation (A) contains a sodium salt of ethylenediaminetetraacetic acid (EDTA).

5. The use according to claim 4, **characterized in that** preparation (A) contains disodium ethylenediaminetetraacetate (Na₂H₂EDTA).

6. The use according to one of claims 1 to 5, **characterized in that** preparation (A) contains, based on its weight, the salt of ethylenediaminetetraacetic acid (EDTA) in an amount greater than 0.6 wt.%, preferably in amounts of from 0.8 to 2.5 wt.%, more preferably from 1.0 to 2.2 wt.%, and particularly preferably from 1.2 to 2.0 wt.%.

7. The use according to one of claims 1 to 6, **characterized in that** the preparation contains at least one persulfate selected from ammonium peroxodisulfate and/or potassium peroxodisulfate and/or sodium peroxodisulfate.

8. The use according to one of claims 1 to 7, **characterized in that** preparation (B) contains, based on its weight, hydrogen peroxide in amounts of from 0.5 to 30 wt.%, preferably from 1 to 20 wt.%, more preferably from 5 to 15 wt.%, and particularly preferably from 6 to 12 wt.%.

## Revendications

1. Utilisation d'un polysaccharide et d'un sel d'acide éthylènediaminetétraacétique (EDTA) dans une préparation (A) faisant partie d'un agent d'éclaircissement de fibres kératiniques, contenant au moins deux préparations (A) et (B) conditionnées séparément et éventuellement une autre préparation (C) conditionnée séparément de (A) et (B), lesquelles sont mélangées immédiatement avant utilisation pour former un mélange à appliquer, **caractérisée en ce que** :
i. la préparation (A) contient au moins un persulfate, et **en ce que**
ii. la préparation (B) est fluide et contient au moins un agent oxydant,
pour réduire des dommages causés aux cheveux et/ou au cuir chevelu, et/ou
pour réduire la quantité de chaleur générée lors de l'éclaircissement des fibres kératiniques.

2. Utilisation selon la revendication 1, **caractérisée en ce que** la préparation (A) contient comme polysaccharide un dérivé de cellulose choisi dans le groupe constitué par la méthylcellulose, l'éthylcellulose, la propylcellulose, la méthyléthylcellulose, la carboxyméthylcellulose, l'éthylcarboxyméthylcellulose, l'hydroxyéthylcellulose, l'hydroxypropylcellulose, la carboxyméthylhydroxyéthylcellulose, la méthylhydroxyéthylcellulose, l'hydroxypropylméthylcellulose, l'éthylhydroxyéthylcellulose, la méthyléthylhydroxyéthylcellulose et des mélanges de ces composés.

3. Utilisation selon la revendication 2, **caractérisée en ce que** la préparation (A) contient de la carboxyméthylcellulose comme polysaccharide.

4. Utilisation selon l'une des revendications 1 à 3, **caractérisée en ce que** la préparation (A) contient un sel de sodium de l'acide éthylènediaminetétraacétique (EDTA).

5. Utilisation selon la revendication 4, **caractérisée en ce que** la préparation (A) contient de l'éthylènediaminetétraacétate de disodium (Na₂H₂EDTA).

6. Utilisation selon l'une des revendications 1 à 5, **caractérisée en ce que** la préparation (A) contient, par rapport à son poids, du sel d'acide éthylènediaminetétraacétique (EDTA) en une quantité supérieure à 0,6 % en poids, de préférence de 0,8 à 2,5 % en poids, de manière davantage préférée de 1,0 à 2,2 % en poids et de façon tout particulièrement préférée de 1,2 à 2,0 % en poids.

7. Utilisation selon l'une des revendications 1 à 6, **caractérisée en ce que** la préparation (A) contient au moins un persulfate choisi parmi le peroxodisulfate d'ammonium et/ou de potassium et/ou le peroxodisulfate de sodium.

8. Utilisation selon l'une des revendications 1 à 7, **caractérisée en ce que** la préparation (B) contient, par rapport à son poids, du peroxyde d'hydrogène en des quantités de 0,5 à 30 % en poids, de préférence de 1 à 20 % en poids, de préférence de 5 à 15 % en poids et de façon tout particulièrement préférée de 6 à 12 % en poids.
